# EUROPEAN PATENT APPLICATION

(11) **EP 4 487 859 A1**
(43) Date of publication of application: **08.01.2025**
(21) Application number: 22929697.5
(22) Date of filing: 01.03.2022
(51) Int. Cl.: A61K 35/747, A23L 33/135, A61P 15/12

(54) **AGENT FOR AMELIORATING SYMPTOMS OF FEMALE MENOPAUSE**

(71) Applicant: Asahi Group Holdings, Ltd., Tokyo 130-8602 (JP)
(72) Inventor: SAWADA Daisuke, Moriya-shi, Ibaraki 302-0106 (JP); SUGAWARA Tomonori, Moriya-shi, Ibaraki 302-0106 (JP)
(74) Representative: J A Kemp LLP
(86) International application number: PCT/JP2022/008548
(87) International publication number: WO 2023/166548

(57) **Abstract**

It is an object to provide a novel technology that can improve a female menopausal symptom. Provided is an agent for improving a female menopausal symptom, containing a lactobacillus strain that is *Lactobacillus gasseri* CP2305 strain (accession number: FERM BP-11331), a processed product of the lactobacillus strain, or an extract thereof.

## Description

### Technical Field

The present invention relates to an agent for improving a female menopausal symptom, containing a lactobacillus strain that is *Lactobacillus gasseri* CP2305 strain, a processed product of the lactobacillus strain, or an extract thereof.

### Background Art

Women reach menopause at about age 50 in average. The period from 10 years before menopause to 10 years after menopause (age 40 to 60) is called the menopausal stage. In the female menopausal stage, various factors such as decline of ovarian function, psychological factors, and environmental factors are complicatedly overlapped to sometimes develop a menopausal symptom (hereinafter referred to as "a female menopausal symptom"). The female menopausal symptom is a physical and/or mental discomfort, which usually continues in years.

To improve such a female menopausal symptom, various techniques have been proposed. For example, Patent Literature 1 proposes a composition for improving a female menopausal symptom which contains an extract from *Pueraria thomsonii* as an active ingredient.

### Citation List

### Patent Literature

Patent Literature 1: Japanese Translation of PCT International Application Publication No. 2020-509989

### Summary of Invention

### Technical Problem

An object of the present invention is to provide a novel technology that can improve a female menopausal symptom.

### Solution to Problem

The present inventors found that a female menopausal symptom can be improved by taking (administering) a lactobacillus strain that is *Lactobacillus gasseri* CP2305 strain, a processed product of the lactobacillus strain, or an extract thereof and accomplished the present invention.

The summary of the present invention is as follows.
[1] An improving agent for improving a female menopausal symptom, containing a lactobacillus strain that is *Lactobacillus gasseri* CP2305 strain (accession number: FERM BP-11331), a processed product of the lactobacillus strain, or an extract thereof.
[2] The improving agent according to [1], wherein the female menopausal symptom is a mental symptom and/or a physical symptom.
[3] The improving agent according to [2], wherein the mental symptom is one or more symptoms selected from the group consisting of insomnia, anger, irritability, and depression.
[4] The improving agent according to [2] or [3], wherein the physical symptom is one or more symptoms selected from the group consisting of getting chilled, headache, dizziness, nausea, fatigue, stiff shoulders, lower back pain, and pain in a limb.
[5] The improving agent according to any one of [1] to [4] to be orally taken.
[6] A food and drink composition for improving a female menopausal symptom, containing a lactobacillus strain that is *Lactobacillus gasseri* CP2305 strain (accession number: FERM BP-11331), a processed product of the lactobacillus strain, or an extract thereof.
[7] A method for improving a female menopausal symptom, comprising administering a lactobacillus strain that is *Lactobacillus gasseri* CP2305 strain (accession number: FERM BP-11331), a processed product of the lactobacillus strain, or an extract thereof.
[8] The method according to [7], wherein the female menopausal symptom is a mental symptom and/or a physical symptom.
[9] The method according to [8], wherein the mental symptom is one or more symptoms selected from the group consisting of insomnia, anger, irritability, and depression.
[10] The method according to [8] or [9], wherein the physical symptom is one or more symptoms selected from the group consisting of getting chilled, headache, dizziness, nausea, fatigue, stiff shoulders, lower back pain, and pain in a limb.
[11] The method according to any one of [7] to [10], comprising orally administering the lactobacillus strain, a processed product of the lactobacillus strain, or an extract thereof.
[12] Use of a lactobacillus strain that is *Lactobacillus gasseri* CP2305 strain (accession number: FERM BP-11331), a processed product of the lactobacillus strain, or an extract thereof for improving a female menopausal symptom.
[13] Use of a lactobacillus strain that is *Lactobacillus gasseri* CP2305 strain (accession number: FERM BP-11331), a processed product of the lactobacillus strain, or an extract thereof for producing an agent for improving a female menopausal symptom.
[14] A therapeutic and/or prophylactic agent for a female menopausal disorder, comprising a lactobacillus strain that is *Lactobacillus gasseri* CP2305 strain (accession number: FERM BP-11331), a processed product of the lactobacillus strain, or an extract thereof.
[15] The therapeutic and/or prophylactic agent according to [14] to be orally taken.
[16] Use of a lactobacillus strain that is *Lactobacillus gasseri* CP2305 strain (accession number: FERM BP-11331), a processed product of the lactobacillus strain, or an extract thereof for treating and/or preventing a female menopausal disorder.
[17] Use of a lactobacillus strain that is *Lactobacillus gasseri* CP2305 strain (accession number: FERM BP-11331), a processed product of the lactobacillus strain, or an extract thereof for producing a therapeutic and/or prophylactic agent for a female menopausal disorder.

### Advantageous Effect of Invention

According to the present invention, it is possible to provide a novel technology that can improve a female menopausal symptom.

### Brief Description of Drawings

[Figure 1] Figure 1 is a graph showing the relationship between a change in SMI score and time (menstrual cycle).
[Figure 2] Figure 2 is a graph showing the relationship between a change in mental symptom score and time (menstrual cycle) (Figure 2 (a)), and a graph showing the relationship between a change in physical symptom score and time (menstrual cycle) (Figure 2 (b)).

### Description of Embodiments

Now, an embodiment of the present invention will be described.

The embodiment relates to an agent for improving a female menopausal symptom (hereinafter also referred to simply as "improving agent"). The improving agent according to the embodiment contains a lactobacillus strain that is *Lactobacillus gasseri* CP2305 strain (hereinafter also referred to simply as "CP2305 strain") a processed product of CP2305 strain, or an extract thereof.

First, the CP2305 strain for use in the improving agent according to the embodiment will be specifically described. Note that, in the following explanation, the CP2305 strain, a processed product thereof, and an extract thereof will be also collectively referred to as a CP2305 strain, or the like.

*Lactobacillus gasseri* CP2305 strain for use in the improving agent according to the embodiment is a type of lactobacillus strain and deposited as of September 11, 2007 at the Patent Organism Depositary of the National Institute of Advanced Industrial Science and Technology, which is the International Depositary Authority under the Budapest Treaty provision (Central 6, 1-1, Higashi 1-chome Tsukuba-shi, Ibaraki-ken 305-8566, Japan). The international deposit accession number is FERM BP-11331. Note that, the service for the patent microorganism deposit as the International Depositary Authority under the Budapest Treaty at the Patent Organism Depositary of the National Institute of Advanced Industrial Science and Technology has been succeeded by the Patent Organism Depositary of the National Institute of Technology and Evaluation (#120, 2-5-8 Kazusakamatari, Kisarazu-shi, Chiba 292-0818, Japan) on April, 2012.

CP2305 strain to be used in an improving agent according to the embodiment may be cultured in any medium and the medium is not particularly limited as long as CP2305 strain can grow. For example, CP2305 strain cultured in a medium commonly used for culturing a lactobacillus bacterium (for example, a synthetic medium such as MRS medium) or CP2305 strain cultured in a modified medium thereof may be used.

The nutrition to be used in a medium for culturing CP2305 strain is not particularly limited as long as CP2305 strain can grow.

Examples of a carbon supply-source (hereinafter also referred to as a "carbon source") include glucose, sucrose, lactose, and molasses, which are usually used for culture of a microorganism. These carbon sources may be used in combination of two types or more. Examples of a nitrogen supply-source (hereinafter also referred to as a "nitrogen source") include peptone, casein, a casein digest, whey, and a whey digest. These nitrogen sources may be used in combination of two types or more.

To the medium for culturing CP2305 strain, nutrient supply-sources other than the carbon sources and nitrogen sources described above may be added. Examples of such nutrient supply-sources may include corn steep liquor (CSL), a yeast extract, a meat extract, a liver extract, and a tomato juice. These supply-sources may be used in combination of two types or more.

To a medium for culturing CP2305 strain, components other than the nutrient supply-sources described above, such as a reducing agent, a growth-promoting factor, and a substance capable of imparting a buffer capacity may be added. Examples of the reducing agent may include L-cysteine. Examples of the growth-promoting factor may include vitamins, nucleic acid-related substances, acetates, citrates, fatty acid esters, and Tween 80. Of these, Tween 80 is more preferable. Examples of the substance capable of imparting the buffer capacity may include phosphates.

A method for culturing CP2305 strain is not particularly limited as long as CP2305 strain can grow; for example, static culture and neutralization culture keeping pH to be controlled constant can be used.

In a case where CP2305 strain is cultured in a medium, bacterial cells of the CP2305 strain collected from a culture can be used as an improving agent according to the embodiment. As the method for collecting the bacterial cells, which is not particularly limited to, for example, centrifugation and membrane filtration can be used.

The improving agent according to the embodiment, as mentioned above, contains CP2305 strain, a processed product of CP2305 strain (hereinafter also referred to simply as a "processed product"), and an extract of CP2305 strain and/or a processed product thereof (hereinafter also referred to simply as an "extract"). In other words, the improving agent according to the embodiment contains at least one or more selected from CP2305 strain, the processed product, and the extract.

As the CP2305 strain that can be contained in the improving agent according to the embodiment, for example, living cells, wet bacteria, and dried bacteria of the CP2305 strain can be used.

As the processed product that can be contained in the improving agent according to the embodiment, for example, a processed product obtained by physically processing CP2305 strain and a processed product obtained by chemically processing CP2305 strain can be used. Specific examples of the processed product include a concentrate of fermented milk containing CP2305 strain, a paste, dried material, liquid, dilution, and crushed material thereof. Note that, as the dried material, one dried material selected from the group consisting of a spray-dried material, a freeze-dried material, a vacuum-dried material, and a drum-dried material can be used.

The CP2305 strain in the processed product may be alive or dead. The dead bacterial cells can be obtained usually by heating bacterial cells. The heating condition is not particularly limited as long as bacterial cells are killed. Bacterial cells are generally killed by heating them at 105°C for about 30 minutes. A method for obtaining dead bacterial cells is not limited to the heating method mentioned above and a method of killing bacterial cells by radiation and a method of killing bacterial cells by grinding can be used.

As the extract that can be contained in the improving agent according to the embodiment, an extract obtained by extracting CP2305 strain with a solvent can be used. Examples of the solvent to be used for extraction can include, but are not particularly limited to, chloroform, ethyl acetate, hexane, diethyl ether, dimethyl sulfoxide, methanol, ethanol, water, or a mixed solvent of these. Extraction conditions such as extraction temperature and extraction time are not particularly limited and conditions commonly employed for extracting bacterial cells can be used.

The dosage form (formulation) of the improving agent according to the embodiment is not particularly limited and all dosage forms employed in, e.g., medicines, quasi-drugs, or foods and drinks can be used. Note that, the method for taking (administering) the improving agent according to the embodiment can be appropriately set depending on the dosage form. Although it is not particularly limited, an oral intake (administration) method can be employed.

The improving agent according to the embodiment may contain not only CP2305 strain, or the like. but also other components excluding CP2305 strain, or the like. For example, if the improving agent according to the embodiment is a medicine, a quasi-drug, or a food and drink, the improving agent according to the embodiment may contain not only CP2305 strain, or the like but also other components such as an excipient, a binding agent, a stabilizer, a disintegrant, a lubricant, a flavoring agent, a suspension agent, a coating agent, a food, and a beverage.

The dosage form of the improving agent according to the embodiment is not particularly limited and can be appropriately set depending on the dosage form (formulation) of the improving agent. For example, if the improving agent according to the embodiment is a medicine, a quasi-drug, or a food and drink, the dosage form can be, e.g., a tablet, a pill, a capsule, a granule, a powder, or a syrup.

In particular, if the improving agent according to the embodiment is a food and drink, the improving agent may be prepared as a common food and drink but as food for special uses such as food for specified health uses or a food with nutrient function claims. Specific examples of the food and drink may include nutritional supplements, milks, processed milks, milk drinks, soft drinks, alcoholic beverages, non-alcoholic beers, fermented milks, yogurts, cheeses, breads, biscuits, crackers, pizza crusts, ice creams, candies, gummies, gums, modified milk powders, fluid diets, diets for patients, foods such as powdered milks for infants, and foods such as powdered milks for nursing mothers.

The improving agent according to the embodiment can be produced by appropriately mixing CP2305 strain, or the like, and the aforementioned components to be optionally added in accordance with a routine method. The production method and conditions are not particularly limited.

The content of CP2305 strain, or the like in the improving agent according to the embodiment is not particularly limited. For example, in the case of adults, the content can be set such that 100 million to 100 billion of bacterial cells of CP2305 strain (or an equivalent amount of a processed product of CP2305 strain, an equivalent amount of an extract of CP2305 strain) can be taken. The period for taking the improving agent according to the embodiment is not particularly limited and can be, for example, the period of one or more menstrual cycles (in other words, continuously 4 weeks or more), the period of two or more continuous menstrual cycles (in other words, continuously 8 weeks or more), or the period of 6 or more continuous menstrual cycles (in other words, continuously 24 weeks or more).

When the improving agent according to the embodiment as described above is taken by a person having a female menopausal symptom, the female menopausal symptom can be improved. Note that, the phrase "the female menopausal symptom can be improved" means that the degree (severity) of a female menopausal symptom can be reduced by taking the improving agent according to the embodiment, compared to the case where the improving agent according to the embodiment is not taken, but does not necessarily mean that a female menopausal symptom does not occur.

The female menopausal symptom to be improved by taking the improving agent according to the embodiment is at least one symptom selected from the group consisting of mental symptoms, physical symptoms, and vasomotor symptoms, and is a symptom that appears in women of 40 to 60 years old (40 years old or more to 60 years old or less). A female menopausal symptom is a symptom usually not caused by an organic change and continues in years (for example, one year or more). Since a female menopausal symptom usually continues in years, the symptom is distinguished from the symptom that appears before menstruation and reduces or disappears during menstruation such as a premenstrual syndrome (PMS) and a premenstrual dysphoric disorder (PMDD). The female menopausal symptom to be improved by the improving agent according to the embodiment is preferably a mental symptom and/or a physical symptom since these symptoms are more easily improved.

A mental symptom among the female menopausal symptoms is a psychological symptom such as insomnia (e.g., sleep onset disorder, sleep deep disorder), anger, irritability, depression, and anxiety. The mental symptom to be improved by the improving agent according to the embodiment is preferably one or more symptoms selected from the group consisting of insomnia, anger, irritability, and depression since it is more easily improved.

Note that, anger can be defined as hyperactivity of emotional excitation, that is, a state where a person gets in a bad mood due to a trifling matter and deep emotion becomes hyperactive, according to a published document (Standard Psychiatry 6th edition (Igaku-Shoin)). Irritability can be defined as the state where things do not go as a person expected and the person becomes anxious, according to the Japanese Society of Psychosomatic Obstetrics and Gynecology. Depression is synonymous with depressive mood and can be defined as being in a sinking feeling without no reason, according to the published document (Standard Psychiatry 6th edition (Igaku-Shoin)). Anxiety can be defined as feeling of objectless fear according to the published document (Standard Psychiatry 6th edition (Igaku-Shoin)).

A physical symptom among the female menopausal symptoms, refers to a distressing symptom excluding vasomotor symptoms. Examples of specific symptoms may include getting chilled, headache, dizziness, nausea, fatigue, stiff shoulders, lower back pain, pain in the limb (joint pain), thirst, swelling, numbness, diarrhea, and constipation. The physical symptom to be improved by the improving agent according to the embodiment is preferably one or more symptoms selected from the group consisting of getting chilled, headache, dizziness, nausea, fatigue, stiff shoulders, lower back pain, and pain in the limb since it is more easily improved. Note that, fatigue is also called as tiredness and can be defined as the state of decreased physical activity with discomfort caused by excessive physical and mental activity or disease, and a desire of rest, according to the Japanese Society of Fatigue.

A vasomotor symptom among the female menopausal symptoms is a physical symptom in which the vasomotor nerve is involved and a symptom caused by e.g., acceleration of metabolism. Examples of specific symptom include, rush of blood to head, hot flashes, sweating, short breath, and palpitations.

If a woman of 40 to 60 years old has at least one of the aforementioned symptoms, it may be determined that the woman has a female menopausal symptom. Preferably, if a woman has a simplified menopausal index (Simplified Menopausal Index (SMI)) score of 26 or more, it can be determined that the woman has a female menopausal symptom. The upper limit of the simplified menopausal index (SMI) may be 100 or less and is not particularly limited. For example, if the SMI score is 80 or less (26 or more and 80 or less), it may be determined that the woman has a female menopausal symptom. If the SMI score is 65 or less (26 or more and 65 or less), it may be determined that the woman has a female menopausal symptom. If the SMI score is 50 or less (26 or more and 50 or less), it may be determined that the woman has a female menopausal symptom.

Note that, the simplified menopausal index (SMI) is an index based on which the presence or absence and severity of a menopausal symptom are determined and obtained by evaluating the presence or absence and severities of 10 symptoms regarding mental symptoms, physical symptoms, and vasomotor symptoms and expressing the evaluation results as scores. Simplified menopausal index are more specifically described in, for example, a published document (Women's Medical Guidebook, Menopausal Medical Edition, 2014 annual edition (the Japan Society for Menopause and Women's Health)).

It is satisfactory that a person taking the improving agent according to the embodiment is at least a woman of 40 to 60 years old having a female menopausal symptom. The person may be a premenopausal woman or a postmenopausal woman but is preferably a premenopausal woman. Note that menopause can be defined as the state where the ovarian gradually becomes inactive to stop menstruation permanently according to the Japan Society of Obstetrics and Gynecology. The onset of menopause can be determined, after no menstruation occurs during continuous 12 months or more, as the onset of menopause was a year before. Menopause can be determined based on the blood concentrations of estradiol (E2) and follicle-stimulating hormone (FSH) as criteria. For example, if the blood concentration of E2 during the follicular phase exceeds 10 pg/ml, the woman is determined to be premenopausal (in other words, if the blood concentration of E2 is 10 pg/ml or less, the woman is determined to be postmenopausal). Furthermore, for example, if the blood concentration of FSH during the follicular phase is below 40 mIU/ml, the woman is determined to be premenopausal (in other words, if the blood concentration of FSH is 40 mIU/ml or more, the woman is determined to be postmenopausal).

In the aforementioned improving agent according to the embodiment, CP2305 strain, or the like are used for improving a female menopausal symptom and for producing an agent for improving a female menopausal symptom. More specifically, as an aspect of the embodiment includes use of CP2305 strain, or the like for improving a female menopausal symptom (use of a lactobacillus strain that is *Lactobacillus gasseri* CP2305 strain, a processed product of the lactobacillus strain, or an extract thereof) and use of CP2305 strain, or the like for producing an agent for improving a female menopausal symptom (use of a lactobacillus strain that is *Lactobacillus gasseri* CP2305 strain, a processed product of the lactobacillus strain or an extract thereof). Note that since CP2305 strain, or the like to be used for improving a female menopausal symptom and CP2305 strain, or the like to be used for producing an agent for improving a female menopausal symptom have the same constitution as in the CP2305 strain, or the like of the aforementioned improving agent, a detailed explanation is omitted.

Furthermore, the improving agent according to the embodiment can improve a female menopausal symptom, as mentioned above. Because of this, the improving agent according to the embodiment can be used as a therapeutic agent and/or prophylactic agent for a female menopausal disorder. More specifically, according to the present invention, as another embodiment different from an embodiment of the improving agent, it is possible to provide a therapeutic and/or prophylactic agent (hereinafter also referred to as "therapeutic/prophylactic agent") for a female menopausal disorder, containing a lactobacillus strain that is *Lactobacillus gasseri* CP2305 strain (accession number: FERM BP-11331), a processed product of the lactobacillus strain, or an extract thereof. Note that, since CP2305 strain, or the like to be contained in the therapeutic/prophylactic agent have the same constitution as in the CP2305 strain, or the like contained in the aforementioned improving agent, a detailed explanation is omitted. The female menopausal disorder herein refers to a state where daily life is impaired by at least one of the female menopausal symptoms.

In the aforementioned embodiment of a therapeutic/prophylactic agent, CP2305 strain, or the like are used for treating or preventing a female menopausal disorder and for producing a therapeutic/prophylactic agent for a female menopausal disorder. More specifically, an aspect of the embodiment of the therapeutic/prophylactic agent includes use of CP2305 strain, or the like for treating and/or preventing a female menopausal disorder (use of a lactobacillus strain that is *Lactobacillus gasseri* CP2305 strain, a processed product of the lactobacillus strain, or an extract thereof), and use of CP2305 strain, or the like for producing a therapeutic and/or prophylactic agent for a female menopausal disorder (use of a lactobacillus strain that is *Lactobacillus gasseri* CP2305 strain, a processed product of the lactobacillus strain, or an extract thereof). Note that, since CP2305 strain, or the like to be used for treating and/or preventing a female menopausal disorder and CP2305 strain, or the like to be used for producing a therapeutic and/or prophylactic agent for a female menopausal disorder have the same constitution as in the CP2305 strain, or the like contained in the aforementioned therapeutic/prophylactic agent, a detailed explanation is omitted.

### Examples

Now, the present invention will be described more specifically by way of Examples but the present invention is not limited to these.

### [Experiment 1 (menopausal symptoms)]

Heat-killed bacterial cells of CP2305 strain, maltose (excipient), dextrin (binding agent), starch (binding agent), and a vegetable oil (lubricant) were mixed. The obtained mixture was compressed into round tablets (compression molding) of 8 mm in diameter (hereinafter also referred to as "CP2305 tablets"). A single CP2305 tablet had a weight of 0.22 g containing about 5 billion heat-killed bacterial cells of CP2305 strain.

Furthermore, round tablets of 8 mm in diameter (hereinafter also referred to as "Placebo tablets") were obtained in the same manner as in producing CP2305 tablets except that heat-killed bacterial cells of CP2305 strain were not used and the amount of maltose was increased (to the same amount as the heat-killed bacterial cells). A single Placebo table had the same weight (0.22 g) as the single CP2305 tablet.

Using CP2305 tablets and Placebo tablets (these will be hereinafter also referred to as "tablets"), a randomized placebo-controlled trial (RCT) was performed in accordance with a double-blind test, as shown below.

First, to choose test subjects, simplified menopausal indexes (hereinafter also referred to simply as "SMI") on 40 to 59 year-old premenopausal healthy women (humans) were obtained. More specifically, 40 to 59 year-old premenopausal healthy women were asked to evaluate the severities of 10 menopausal symptoms shown the following Table 1 as 4 stages ("none", "light", "medium", "severe") based on a published document (Women's Medical Guidebook, Menopausal Medical Edition, 2014 (the Japan Society for Menopause and Women's Health)). The evaluation results are indicated by scores in accordance with the following Table 1 and the total score was obtained and used as SMI. The self-evaluations were all carried out during the period from the onset of menstruation to Day 6 (0th menstruation) to avoid the effects of female hormones due to menstruation.

Note that, menopausal symptoms to be evaluated in the following Table 1 are 10 symptoms including vasomotor symptoms (symptoms 1, 2, 4 shown in Table 1), mental symptoms (symptoms 5 to 7 shown in Table 1), and physical symptoms (symptoms 3, 8 to 10 shown in Table 1). Before self-evaluation was started, to perform evaluation on the same criteria, the following definitions on the severities of symptoms were presented to test subjects.

### [Definition]

Severity of symptom "none": No symptom.

Severity of symptom "light": Symptom is recognized but does not affect daily life.

Severity of symptom "medium": Symptom is severe, sometimes unbearable.

Severity of symptom "severe": Symptom is very severe and affects daily life.

**[Table 1]**

| Symptom | | | Severity of symptom (score) | | | |
|---|---|---|---|---|---|---|
| | | | Severe | Medium | Light | None |
| 1 | Vasomotor symptom | Face feels hot | 10 | 6 | 3 | 0 |
| 2 | | Easily sweat | 10 | 6 | 3 | 0 |
| 3 | Physical symptom | Lower back and limb easily get cold | 14 | 9 | 5 | 0 |
| 4 | Vasomotor symptom | Shortness of breath and palpitations | 12 | 8 | 4 | 0 |
| 5 | Mental symptom | Having trouble falling asleep or light sleep | 14 | 9 | 5 | 0 |
| 6 | | Easily angered and easily irritated | 12 | 8 | 4 | 0 |
| 7 | | Sometimes worry or feel depressed | 7 | 5 | 3 | 0 |
| 8 | Physical symptom | Often has headache, dizziness, nausea | 7 | 5 | 3 | 0 |
| 9 | | Easily get tired | 7 | 4 | 2 | 0 |
| 10 | | Stiff shoulders, lower back pain, and pain in a limb | 7 | 5 | 3 | 0 |

After SMI scores were obtained in the aforementioned manner, 94 women having an SMI score of 26 to 50 were extracted, and the 94 women were determined as test subjects having menopausal symptoms (menopausal symptoms with a severity not affecting daily life). Note that, the subjects all had a beyond 10 pg/ml blood concentration of estradiol (E2) in the follicular phase and a below 40 mIU/ml blood concentration of follicle-stimulating hormone (FSH) in the follicular phase.

The test subjects (94 women) were divided into two groups: a group of taking a CP2305 tablet (hereinafter also referred to as "CP2305 group") and a group of taking a Placebo tablet (hereinafter also referred to as a "Placebo group"). Tablets were continuously taken at a dose of 2 tablets per day in the period of consecutive 6 menstrual cycles. Note that the intake of tablets was initiated in a unified manner on the day of onset of menstruation (0th menstruation), on which self-evaluation (self-evaluation for obtaining SMI) was made.

The period from the onset of menstruation on which intake of a tablet was initiated to the onset of the next menstruation (1st menstruation) was determined as the first menstrual cycle. SMI was obtained immediately after completion of the second menstrual cycle, fourth menstrual cycle, and sixth menstrual cycle, three times in total, in the same manner as mentioned above.

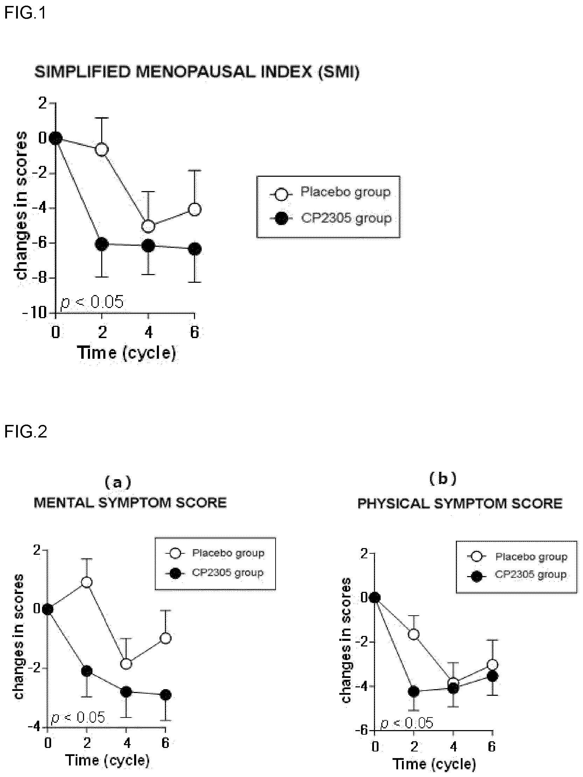

The results are shown in Figure 1. Note that, in Figure 1, the vertical axis represents a change in SMI score (average value) based on the SMI (SMI obtained when test subjects were chosen) before initiation of tablet intake. A case where a change in SMI score is lower than 0 indicates that SMI is low compared to that before initiation of tablet intake. The horizontal axis represents time (menstrual cycle).

As is apparent from Figure 1, the change in SMI score of the CP2305 group was continuously lower than 0 during consecutive 6 menstrual cycles after tablet intake. Furthermore, the change in SMI score of the CP2305 group during consecutive 6 menstrual cycles after tablet intake was continuously low even compared to that of the Placebo group. From the statistical analysis using repeated measures analysis of variance (Repeated Measures ANOVA), it was confirmed that the change in SMI score was preferentially low. From the result, it could be confirmed that the menopausal symptoms of the CP2305 group could be improved.

Next, from SMI scores immediately after completion of the second cycle, the fourth cycle, and the sixth cycle, only scores on the mental symptoms (symptoms 5 to 7 shown in Table 1) were extracted and summed up per every menstrual cycle (hereinafter also referred to as the "mental symptom score"). Similarly, only scores on the physical symptoms (symptoms 3, 8 to 10 shown in Table 1) were extracted from SMI scores immediately after completion of the second cycle, the fourth cycle, and the sixth cycle and summed up per every menstrual cycle (hereinafter also referred to as "physical symptom score").

The results of the mental symptom scores are shown in Figure 2 (a) and the results of the physical symptom scores are shown in Figure 2 (b).

Note that, in Figure 2 (a), the vertical axis represents a change in score (average value) based on the mental symptom score (mental symptom score extracted from the SMI scores obtained at the time the test subjects were chosen) before initiation of tablet intake. A case where a change in score is lower than 0 indicates that mental symptom score is low compared to that before the initiation of tablet intake. In Figure 2 (b), the vertical axis represents a change in score (average value) based on the physical symptom score (physical symptom score extracted from the SMI scores obtained at the time the test subjects were chosen) before initiation of tablet intake. A case where a change in score is lower than 0 indicates that physical symptom score is low compared to that before the initiation of tablet intake. In Figure 2 (a) and Figure 2 (b), the horizontal axis represents time (menstrual cycle).

As is apparent from Figure 2 (a) and Figure 2 (b), changes in mental symptom score and physical symptom score were continuously lower than 0 during consecutive 6 menstrual cycles after tablet intake in the CP2305 group. Changes in mental symptom score and physical symptom score were continuously lower during consecutive 6 menstrual cycles after tablet intake in the CP2305 group even compared to those of the Placebo group. From the statistical analysis using repeated measures analysis of variance (Repeated Measures ANOVA), it was confirmed that these changes in score were preferentially low. From these results, it could be confirmed that, in the CP2305 group, mental symptoms (symptom 5 (insomnia), symptom 6 (anger, irritability), and symptom 7 (depression)) among the menopausal symptoms could be improved, and physical symptoms (symptom 3 (getting chilled), symptom 8 (headache, dizziness, nausea), symptom 9 (fatigue), and symptom 10 (stiff shoulders, lower back pain, pain in the limb)) among the menopausal symptoms could be improved.

## Claims

1. An improving agent for improving a female menopausal symptom, comprising a lactobacillus strain that is *Lactobacillus gasseri* CP2305 strain (accession number: FERM BP-11331), a processed product of the lactobacillus strain, or an extract thereof.

2. The improving agent according to claim 1, wherein the female menopausal symptom is a mental symptom and/or a physical symptom.

3. The improving agent according to claim 2, wherein the mental symptom is one or more symptoms selected from the group consisting of insomnia, anger, irritability, and depression.

4. The improving agent according to claim 2 or 3, wherein the physical symptom is one or more symptoms selected from the group consisting of getting chilled, headache, dizziness, nausea, fatigue, stiff shoulders, lower back pain, and pain in a limb.

5. The improving agent according to any one of claims 1 to 4 to be orally taken.

6. A food and drink composition for improving a female menopausal symptom, comprising a lactobacillus strain that is *Lactobacillus gasseri* CP2305 strain (accession number: FERM BP-11331), a processed product of the lactobacillus strain, or an extract thereof.
